# EUROPÄISCHE PATENTSCHRIFT

(11) **EP 2 897 672 B1**
(45) Veröffentlichungstag und Bekanntmachung des Hinweises auf die Patenterteilung: **09.01.2019**
(21) Anmeldenummer: 13815694.8
(22) Anmeldetag: 18.12.2013
(51) Int. Cl.: A61M 5/145, A61M 5/168

(54) **DAUERINFUSIONSVORRICHTUNG**
CONTINUOUS INFUSION DEVICE
DISPOSITIF DE PERFUSION CONTINUE

(30) Priorität: 16.01.2013 DE 202013000411 U
(43) Veröffentlichungstag der Anmeldung: 29.07.2015
(73) Patentinhaber: H & B Electronic GmbH & Co. KG, 75392 Deckenpfronn (DE)
(72) Erfinder: WEBER, Wilfried, 72296 Schopfloch (DE); MORLOK, Tobias, 71159 Mötzingen (DE)
(74) Vertreter: Wacker, Jost Oliver
(86) Internationale Anmeldenummer: PCT/EP2013/003825
(87) Internationale Veröffentlichungsnummer: WO 2014/111114

(56) Entgegenhaltungen:
- EP-A1- 0 016 343
- EP-A1- 0 598 678
- EP-A1- 3 028 727
- WO-A1-2012/120765
- DE-A1- 10 004 496
- US-A- 2 602 446
- US-A- 4 273 122
- US-A- 4 430 079
- US-A1- 2003 100 863

## Beschreibung

Die Erfindung betrifft eine Vorrichtung für Dauerinfusionen, wie beispielsweise zur Verabreichung von Medikamenten gegen Multiple Sklerose nach dem Oberbegriff des Anspruchs 1. Diese Dauerinfusionsvorrichtung weist ein Gehäuse auf, in dem ein Infusionsgerät mit wenigstens einem auspressbaren Infusionsflüssigkeitsbehälter eingesetzt werden kann. Zudem sind in der Dauerinfusionsvorrichtung eine Betätigungsvorrichtung zum Auspressen des Infusionsgerätes und ein Antrieb zur Verlagerung der Betätigungsvorrichtung vorgesehen, der über eine mechanische Kraftspeichereinrichtung mit der notwendigen Antriebsenergie versorgt wird. Darüber hinaus weist die Dauerinfusionsvorrichtung eine mechanische Steuerungsanordnung auf, mittels der eine gewünschte Geschwindigkeit der Betätigungsvorrichtung eingestellt werden kann.

Mit derartigen mechanisch betriebenen Dauerinfusionsvorrichtungen können unabhängig von einem lokal vorhandenen Stromnetz oder einer Batteriespannung kleine Dosen eines Medikaments über mehrere Stunden hinweg kontinuierlich in die Blutbahn eines Patienten abgegeben werden. Dies ermöglicht einen sehr flexiblen und beispielsweise mobilen Einsatz der Vorrichtungen, wobei auch keinerlei Vorsichtsmaßnahmen hinsichtlich eines möglichen Stromausfalls oder einer nicht ausreichenden Batteriespannung während eines Infusionsvorganges erforderlich sind.

EP 3 028 727 A1 beschreibt einen Antriebsmechanismus zur gesteuerten Abgabe von Medikamenten aus einem Arzneimittelbehälter mittels einer Kolbenvorrichtung. Der Kolben wird dabei von einem federförmigen Vorspannelement angetrieben. Ferner ist ein Abgabesteuermechanismus vorgesehen, der ein Bandelement umfasst, das den Kolben mit einer Windentrommel verbindet und dabei der freien Verschiebung des Kolbens durch das Vorspannelement entgegen wirkt, wobei die Bewegung des Kolbens durch einen elektrischen Motor gesteuert wird.

Aus der DE 29 06 830 B1 ist ein Gerät für Dauerinfusionen bekannt, das sowohl über ein mechanisches Vorschubwerk angetrieben ist als auch eine rein mechanische Vorschubsteuerung aufweist. Die Vorschubsteuerung ist hierbei durch ein Uhrwerk gebildet. Das Vorschubwerk und das Uhrwerk werden mittels einer jeweiligen Spiralzugfeder angetrieben, wobei beide Spiralzugfedern beim Öffnen eines Gehäusedeckels gespannt werden.

EP 0 598 678 A1 beschreibt einen Schmierstoffgeber für Maschinen, der über eine Steuervorrichtung gesteuert wird, die in der Art eines elektrischen Uhrwerkes betrieben wird. Mittels eines Elektromagneten der Steuervorrichtung wird dabei die Bewegung eines Kolbens kontrolliert, um einen wiederkehrenden Ausstoß des Schmiermittels zu dosieren.

WO 2012/120765 A1 beschreibt eine medizinische Pumpe zur Durchführung von Dauerinfusionen. Diese weist ein Stellrad zur Einstellung einer Strömungsrate einer Medikamentenlösung auf, dessen Drehgeschwindigkeit und Drehrichtung über eine Anordnung von Permanentmagneten und Magneterfassungssensoren von einer Steuerungseinheit erfasst werden kann. Der Antrieb eines Spritzenkolbens erfolgt dabei über die Steuerungseinheit mittels eines elektrisch gesteuerten Motors.

Nachteilig an den bekannten mechanisch arbeitenden Dauerinfusionsvorrichtungen ist, dass diese insbesondere aufgrund des verwendeten Uhrwerks sehr hohe Herstellungskosten haben und relativ empfindlich gegenüber äußeren Einflüssen sind. Zudem unterliegt das Uhrwerk einem gewissen Verschleiß und muss in gewissen Zeitabständen nachgestellt werden, um eine exakte Funktionsweise sicherstellen zu können.

Die Aufgabe der Erfindung ist es, bei einer gattungsgemäßen Dauerinfusionsvorrichtung die genannten Nachteile zu vermeiden und bei geringen Herstellungskosten eine Steuerungsanordnung zur Verfügung zu stellen, die über eine lange und wartungsfreie Lebensdauer hinweg eine zuverlässige Dauerinfusion gewährleistet.

Diese Aufgabe wird durch eine Dauerinfusionsvorrichtung mit den Merkmalen des Anspruchs 1 gelöst. Dabei weist die Steuerungsanordnung eine Bremsvorrichtung mit wenigstens einem abbremsbaren Bremselement auf, das mit dem Antrieb bewegungsgekoppelt ist. Zudem sind an der Bremsvorrichtung Magnetmittel vorgesehen, mittels denen ein Magnetfeld erzeugt werden kann, das das Bremselement mit einer Bremskraft oder einem Bremsmoment beaufschlagt. Auf diese Weise kann das Bremselement kontaktlos durch die Bremskraft beziehungsweise das Bremsmoment gebremst werden, um hierüber eine Geschwindigkeit oder einen Geschwindigkeitsverlauf des Antriebes festlegen zu können. Hierdurch wird eine besonders lange und wartungsfreie Lebensdauer der Steuerungsanordnung ermöglicht, über die hinweg die jeweiligen Infusionsabläufe exakt mit der jeweils eingestellten Geschwindigkeit durchgeführt werden können. Gemäß der Erfindung sind die Magnetmittel zur Einstellung der Bremskraft gegenüber dem Bremselement in ihrer Position verstellbar. Hierdurch kann die jeweils gewünschte Infusionsdauer besonders einfach und exakt über die Bremsvorrichtung eingestellt werden.

In einer besonders vorteilhaften Ausführungsform ist das Bremselement durch eine mittels des Antriebes rotierbare Bremsscheibe gebildet. Die Bremsvorrichtung kann dabei beispielsweise als Wirbelstrom- oder Hysterese-Bremse ausgebildet sein. Hierdurch kann die Dauerinfusionsvorrichtung, insbesondere bei der Verwendung handelsüblicher Wirbelstrom- oder Hysterese-Bremsvorrichtungen, relativ einfach und kostengünstig hergestellt werden.

Dabei ist es günstig, wenn die Magnetmittel durch mindestens einen zum Bremselement beabstandet gehaltenen Dauermagnet gebildet sind, wodurch die Bremsvorrichtung besonders kostengünstig hergestellt und im Wesentlichen ver-Zudem ist es günstig, wenn die Magnetmittel über ein außerhalb des Gehäuses betätigbares erstes Stellelement verstellbar sind, um eine besonders komfortable Einstellung der jeweils gewünschten Infusionsdauer zu ermöglichen.

Vorteilhafterweise weist die Steuerungsanordnung ferner zwischen dem Antrieb und dem Bremselement ein zwischen mindestens zwei unterschiedlichen Übersetzungen schaltbares Getriebe auf, wodurch sehr unterschiedliche Geschwindigkeiten einstellbar sind, was wiederum eine besonders variable Einsetzmöglichkeit der Dauerinfusionsvorrichtung ermöglicht.

Dabei ist es günstig, wenn das Getriebe durch ein schaltbares Planetengetriebe, wie insbesondere in Form eines Ravigneaux-Satzes gebildet wird, was ein störungsfreies Umschalten zwischen den verschiedenen Gängen ermöglicht.

Zudem ist das Getriebe dabei vorteilhafterweise über ein außerhalb des Gehäuses betätigbares zweites Stellelement umschaltbar. Hierdurch ist über das zweite Stellelement zunächst eine Grobeinstellung und über das erste Stellelement ferner eine Feineinstellung der gewünschten Infusionszeit möglich, was wiederum einerseits einen besonders variablen Einsatz der Infusionsvorrichtung und andererseits eine besonders exakte Einstellung der Infusionszeit beziehungsweise der Infusionsgeschwindigkeit ermöglicht.

Ferner ist es günstig, wenn die Betätigungsvorrichtung einen Schlitten aufweist, der entlang einer mit dem Bremselement drehgekoppelten Spindel verlagerbar ist und an dem eine mit der Spindel kämmende Spindelmutter sowie ein an einen Stößel des Infusionsgerätes anlegbares Beaufschlagungselement vorgesehen ist. Hierdurch wird eine besonders exakte Übertragung des am Bremselement wirkenden Bremsmomentes auf die lineare Bewegung des auf das Infusionsgerät einwirkenden Beaufschlagungselementes ermöglicht.

Dabei ist die mechanische Kraftspeichereinrichtung vorteilhafterweise durch eine von Hand spannbare Konstantkraftfeder gebildet, die an dem Schlitten angreift. Hierdurch ist über die Dauer der Infusionsabläufe hinweg eine besonders gleichmäßige Beaufschlagung des Infusionsgerätes möglich.

Zudem ist es günstig, wenn das Infusionsgerät in einer Infusionsgeräteaufnahme gehalten ist, die vor Aktivierung der Betätigungsvorrichtung des Infusionsflüssigkeitsbehälters gegenüber der Betätigungsvorrichtung verlagerbar ist, um unterschiedliche Füllmengen des Infusionsflüssigkeitsbehälters ausgleichen zu können. Hierdurch wird gewährleistet, dass unabhängig vom im Infusionsgerät aufgenommenen Volumen des Medikamentes, dieses bei Start eines Infusionsvorganges ohne wesentliche Verzögerung in der vorgesehenen Dosis verabreicht wird.

Dabei weist das Gehäuse vorteilhafterweise einen zwischen einer Offenstellung und einer Schließstellung verschwenkbaren Deckel auf, wobei die Infusionsgeräteaufnahme mittels eines Wegaufnehmers verlagerbar ist, der beim Verschwenken des Deckels in die Schließstellung beaufschlagbar ist. Hierdurch wird bei jedem Schließen des Gehäuses automatisch ein Volumentoleranzausgleich vorgenommen, so dass unabhängig vom jeweils aufgenommenen Volumen des Medikamentes über die gesamte Anwendungsdauer eine exakte Dosierung des Medikamentes gewährleistet ist.

Ferner ist es günstig, wenn ein erster Sperrmechanismus vorgesehen ist, der in Abhängigkeit einer Position der Betätigungsvorrichtung zwischen einer Sperrstellung, in der der Deckel in der Schließstellung verriegelbar ist, und einer Passivstellung verstellbar ist, in der der Deckel von der Schließstellung in die Offenstellung verschwenkbar ist. Hierdurch kann ein Öffnen des Gehäuses während eines Infusionsvorganges zuverlässig vermieden werden.

Hierbei ist es zudem günstig, wenn die Betätigungsvorrichtung durch ein von außerhalb des Gehäuses betätigbares Einschaltelement aktivierbar ist und ein zweiter Sperrmechanismus vorgesehen ist, der die Betätigung des Einschaltelementes in der Offenstellung des Deckels blockiert. Auf diese Weise kann insbesondere bei der Vorbereitung eines neuen Infusionsvorganges ein versehentliches Aktivieren der Infusionsvorrichtung im geöffneten Zustand des Gehäuses vermieden werden.

In einer weiteren vorteilhaften Ausführungsform der Infusionsvorrichtung ist ein akustischer Signalgeber vorgesehen, der am Ende einer Verlagerungsbewegung der Betätigungsvorrichtung durch diese aktivierbar ist. Hierdurch kann die Betätigungsvorrichtung selbst dazu verwendet werden, am Ende der Verlagerungsbewegung des Signalgebers auszulösen. Hierdurch kann das Ende der Infusionsanwendung dem betreffenden Patienten besonders exakt und zuverlässig signalisiert werden.

Ferner ist es günstig, wenn der Signalgeber eine Glocke sowie ein in einer vorgespannten Stellung verriegelbares und gegen die Glocke impulsartig verlagerbares Anschlagelement aufweist, wobei die Verriegelung mittels der Betätigungsvorrichtung lösbar ist. Hierdurch wird ein besonders exakt und rein mechanisch arbeitender Signalgeber zur Verfügung gestellt.

In einer hierzu alternativen Ausführungsform ist der Signalgeber durch einen elektronischen Signalgeber gebildet, der mit einem über einen Generator aufladbaren Energiespeicher verbunden ist. Mittels dieses Generators kann der Antrieb wenigstens teilweise abgebremst werden. Hierdurch kann der Bremsvorgang wenigstens teilweise zur Erzeugung von Energie genutzt werden, um mit dieser einen kostengünstigen elektronischen Signalgeber zu versorgen.

Dabei ist es besonders günstig, wenn die Bremsvorrichtung durch den Generator gebildet ist, wodurch die Bremsvorrichtung neben ihrer Bremsfunktion gleichzeitig als Energieversorger des Signalgebers fungiert. Hierdurch kann der Aufbau der Dauerinfusionsvorrichtung deutlich vereinfacht werden, was wiederum eines kostengünstigere Herstellung ermöglicht.

In den Figuren ist eine beispielhafte Ausführungsform der Erfindung dargestellt. Es zeigen:
- Figur 1: eine Ansicht einer Rückseite einer erfindungsgemäßen Dauerinfusionsvorrichtung mit entferntem rückseitigem Gehäuseteil,
- Figur 2: eine perspektivische Ansicht der Rückseite der Dauerinfusionsvorrichtung gemäß Fig. 1 und
- Figur 3: eine Ansicht einer Vorderseite der Dauerinfusionsvorrichtung nach Fig. 1.

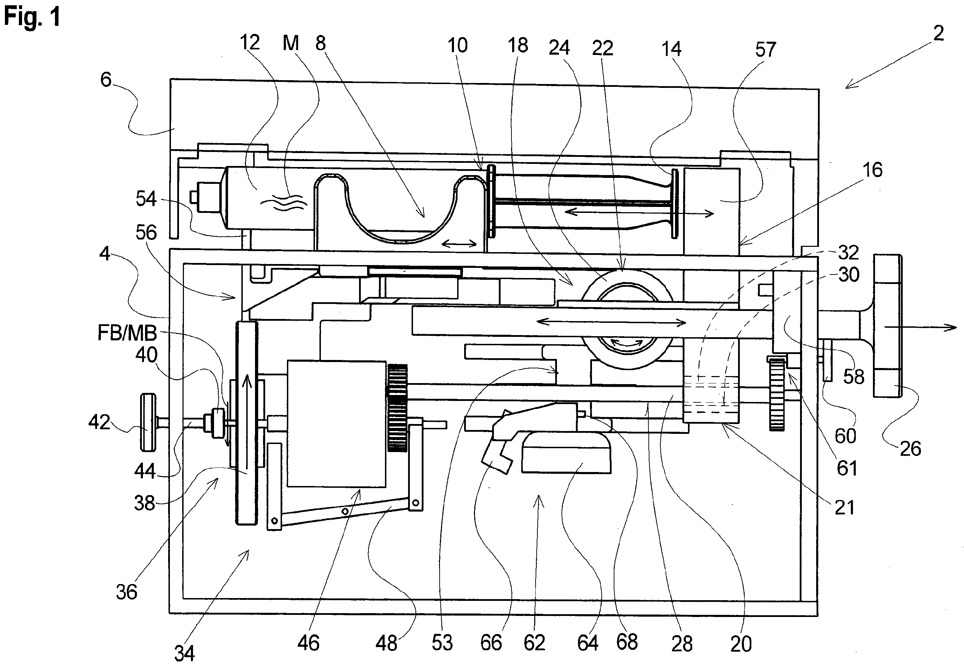

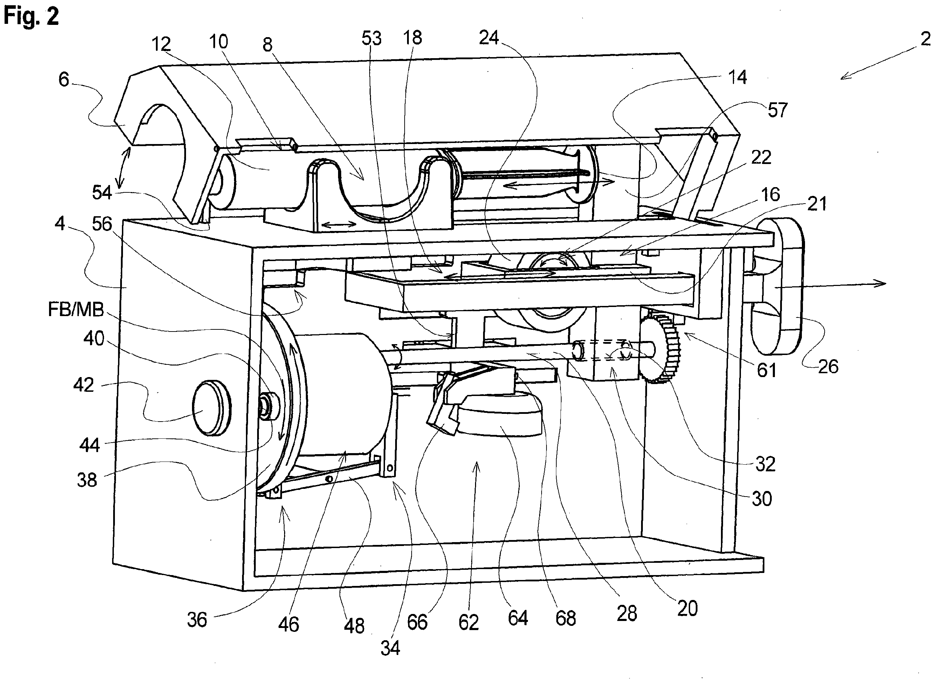

Fig. 1 und 2 zeigen eine Dauerinfusionsvorrichtung 2 mit einem Gehäuse 4, an dem in einem oberen Bereich, der durch einen Deckel 6 verschließbar ist, eine Infusionsgeräteaufnahme 8 aufgenommen ist. In diese Infusionsgeräteaufnahme 8 ist ein spritzenförmiges Infusionsgerät 10 mit einem Infusionsflüssigkeitsbehälter 12 einsetzbar, in dem ein Medium M aufnehmbar ist, das beispielsweise durch ein Medikament zur Behandlung von Multipler Sklerose gebildet ist und durch Beaufschlagung eines Stößels 14 des Infusionsgerätes 10 ausgepresst werden kann.

Zur Beaufschlagung des Stößels 14 ist dabei eine Betätigungsvorrichtung 16 vorgesehen, die mittels eines Antriebs 18 entlang einer Spindel 20 verlagert werden kann. Die Betätigungsvorrichtung 16 weist dabei einen entlang der Spindel 20 verfahrbaren Schlitten 21 auf, an dem eine mechanische Kraftspeichereinrichtung 22 angreift. Diese Kraftspeichereinrichtung 22 weist beispielsweise eine aufrollbare Konstantkraftfeder 24 auf, die mittels eines von außerhalb des Gehäuses 4 handhabbaren Zugelementes 26 aufgezogen werden kann.

Die Spindel 20 bildet hierbei ein Außengewinde 28, das mit einem Innengewinde 30 einer Spindelmutter 32 zusammenwirkt, die in die Betätigungsvorrichtung 16 integriert ist. Ferner ist die Spindel 20 mit einer Steuerungsanordnung 34 gekoppelt, mittels der ein Benutzer der Dauerinfusionsvorrichtung 2 eine gewünschte Infusionsgeschwindigkeit beziehungsweise -zeit einstellen kann.

Die Steuerungsanordnung 34 weist zur Einstellung der gewünschten Infusionsgeschwindigkeit eine Bremsvorrichtung 36 auf. Diese sieht ein Bremselement 38 in Form einer Bremsscheibe vor, die mittels des Antriebes 18 in Rotationsbewegung versetzt werden kann. Das Bremselement 38 wirkt dabei zusammen mit beabstandet gehaltenen Magnetmitteln 40, die beispielhaft durch einen Dauermagneten gebildet sind und ein Magnetfeld erzeugen, das in der Art einer Wirbelstrombremse eine Bremskraft FB beziehungsweise ein Bremsmoment MB am Bremselement 38 generiert. Die Stärke der Bremskraft FB beziehungsweise des Bremsmomentes MB ist dabei abhängig von der Position der Magnetmittel 40 gegenüber dem Bremselement 38, die mittels eines ersten Stellelementes 42 eingestellt werden kann, das von außerhalb des Gehäuses 4 betätigbar und über ein erstes Schaltgestänge 44 mit den Magnetmitteln 40 gekoppelt ist.

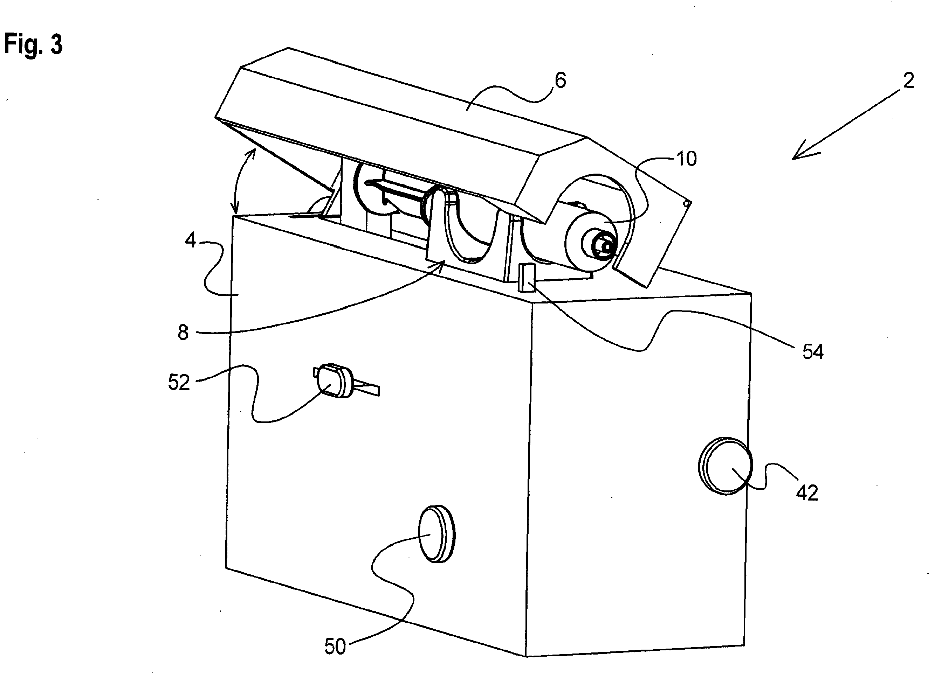

Zudem weist die Steuerungsanordnung 34 zur Einstellung der gewünschten Infusionsgeschwindigkeit ein Getriebe 46 auf, das beispielsweise ein zwischen mindestens zwei Übersetzungen schaltbares Planetengetriebe in Form eines Ravigneaux-Satzes aufweist. Zur Schaltung des Getriebes 46 ist dabei ein zweites Schaltgestänge 48 vorgesehen, das über ein zweites Stellelement 50 von außerhalb des Gehäuses 4 umgeschaltet werden kann, wie aus Fig. 3 zu entnehmen ist.

Am Gehäuse 4 ist zudem ein drittes Stellelement 52 vorgesehen, über das der Patient die benötigte Infusionsmenge des Mediums M einstellen kann. Über das Stellelement 52 ist hierbei ein Anschlagselement 53 in seiner Position verstellbar, das als Endanschlag für die Betätigungsvorrichtung 16 dient.

Wie aus Fig. 3 ferner zu entnehmen ist, ist an dem Gehäuse 4 ein Wegaufnehmer 54 vorgesehen, der vom Deckel 6 beaufschlagt wird, wenn dieser von der dargestellten Offenstellung in eine Schließstellung verschwenkt wird. Der Wegaufnehmer 54 ist dabei Teil eines Toleranzausgleichmechanismus 56, mittels dem die Infusionsgeräteaufnahme 8 soweit gegenüber der Betätigungsvorrichtung 16 verlagerbar ist bis das Infusionsgerät 10 unabhängig von der tatsächlichen Füllmenge des Mediums M mit dem Stößel 14 spielfrei an einem Beaufschlagungselement 57 der Betätigungsvorrichtung 16 anliegt.

Wie aus Fig. 1 zu entnehmen ist, ist im Gehäuse 4 ferner eine Sperrvorrichtung 58 vorgesehen, die einen ersten Sperrmechanismus aufweist, der in Abhängigkeit der jeweils aktuellen Position der Betätigungsvorrichtung 16 zwischen einer Sperrstellung und einer Passivstellung umschaltbar ist. Der erste Sperrmechanismus verhindert dabei ein Verschwenken des Deckels 6 in die Offenstellung, solange die Betätigungsvorrichtung 16 sich nicht in einer Ausgangsstellung befindet, die beispielsweise ihrer Position bei vollständiger Spannung der Kraftspeichereinrichtung 22 entspricht.

Darüber hinaus weist die Sperrvorrichtung 58 einen zweiten Sperrmechanismus auf, der das Einschalten der Dauerinfusionsvorrichtung 2 über ein beispielsweise druckknopfförmiges Einschaltelement 60 gemäß Fig. 1 blockiert, solange sich der Deckel 6 nicht in der Schließstellung befindet. In dieser Blockierstellung verhindert das Einschaltelement 60 über eine mit der Spindel 20 verbundene Sperreinrichtung 61, dass der Antrieb 18 ungewollt aktiviert werden kann.

Als erster und zweiter Sperrmechanismus kann hierbei jede bekannte und geeignete Vorrichtung dienen, wie beispielsweise eine Vorrichtung, die eine verstellbare Sperrklinke und eine entsprechende Klinkenaufnahme aufweist.

Wie aus Fig. 1 und 2 ferner zu entnehmen ist, ist zudem ein akustischer Signalgeber 62 vorgesehen, der eine Glocke 64 und ein Anschlagelement 66 aufweist. Das Anschlagelement 66 ist dabei in der dargestellten gespannten Ausgangsposition der Kraftspeichereinrichtung 22 beispielsweise mittels einer üblichen lösbaren Klinkenvorrichtung gegenüber der Glocke 64 beabstandet gesichert und durch eine Federeinrichtung (nicht dargestellt) in Richtung der Glocke 64 vorgespannt. Die Sicherung des Anschlagselementes 66 in dieser vorgespannten Position kann dabei über ein Auslöseelement 68 aufgehoben werden, das über die Betätigungsvorrichtung 16 beaufschlagt werden kann. Das Auslöseelement 68 ist dabei im Wesentlichen auf Höhe des zur Einstellung der zu verabreichenden Infusionsmenge dienenden Endanschlages angeordnet und hierzu beispielsweise am Anschlagselement 53 gehalten.

Alternativ zu dem mechanisch arbeitenden Signalgeber 62 wäre es auch möglich, einen elektronischen Signalgeber (nicht dargestellt) zu verwenden. Dieser könnte beispielsweise mittels eines Energiespeichers betrieben werden, der über einen Generator aufgeladen wird. Ein solcher Generator könnte dabei gleichzeitig als Teil der Bremsvorrichtung 36 fungieren beziehungsweise direkt durch das Getriebe 46 oder die Spindel 20 angetrieben werden und dadurch ebenfalls mit einem vorzugsweise einstellbaren Bremsmoment MB auf den Antrieb 18 wirken.

Die Funktionsweise der Dauerinfusionsvorrichtung ist dabei in jedem Fall wie folgt:
Zur Vorbereitung eines neuen Infusionsvorganges muss nach der zuletzt vorgenommenen Infusion zunächst die Kraftspeichereinrichtung 22 des Antriebs 18 durch Ziehen des Zugelementes 26 neu gespannt werden, wobei auch die Betätigungsvorrichtung 16 in die dargestellte Ausgangsstellung verbracht wird.

In dieser Ausgangsstellung ist der erste Sperrmechanismus der Sperrvorrichtung 58 in die Passivstellung verbracht, so dass der Patient den Deckel 6 von der Schließstellung in die dargestellte Offenstellung verbringen kann, um ein neues Infusionsgerät 10 mit enthaltenem Medium M in die Infusionsgeräteaufnahme 8 einsetzen zu können.

Anschließend kann der Patient den Deckel 6 wieder in die Schließstellung verschwenken, wobei dieser den Wegaufnehmer 54 beaufschlägt, so dass die Infusionsgeräteaufnahme 8 soweit verlagert wird, bis der Stößel 14 des aufgenommenen Infusionsgerätes 10 an dem Beaufschlagungselement 57 der Betätigungsvorrichtung 16 anliegt. Gleichzeitig wird durch das Verschwenken des Deckels 6 in die Schließstellung der zweite Sperrmechanismus der Sperrvorrichtung 58 von seiner Blockierstellung in eine Passivstellung verbracht, in der das Einschaltelement 60 betätigt werden kann.

Nun kann der Patient über das erste und zweite Stellelement 42, 50 eine gewünschte Infusionsgeschwindigkeit einstellen, wobei das zweite Stellelement 50 beispielsweise zur Einstellung einer groben Geschwindigkeitsabstufung dient, deren Schaltstellungen beispielsweise durch Bezeichnungen wie "schnell", "mittel" und "langsam" angegeben sein können. Das erste Stellelement 42 dient zusätzlich hierzu zu einer Feineinstellung der Infusionsgeschwindigkeit durch Positionierung der Magnetmittel 40.

Darüber hinaus kann der Patient über das dritte Stellelement 52 die Position des Endanschlages für die Betätigungsvorrichtung 16 und damit das zu verabreichende Volumen des Mediums M einstellen.

Hiernach ist die Dauerinfusionsvorrichtung 2 für die vorgesehene Anwendung eingestellt und betriebsbereit.

Bei Druck auf das Einschaltelement 60 löst dieses die Sperreinrichtung 61 an der Spindel 20, so dass diese durch die Rückstellkräfte der an der Betätigungsvorrichtung 16 angreifenden Konstantkraftfeder 24 und dem Zusammenwirken des Innengewindes 30 der Spindelmutter 32 mit dem Außengewinde 28 der Spindel 20 in Rotation versetzt wird. Hierbei wird das Beaufschlagungselement 57 der Betätigungsvorrichtung 16 gegen den Stößel 14 des Infusionsgerätes 10 gedrückt und dadurch das Medium M aus dem Infusionsflüssigkeitsbehälter 12 gepresst, wobei es beispielsweise über einen Schlauch und eine Nadel einem Patienten zugeführt wird (nicht dargestellt).

Durch die Bewegungskoppelung der Spindel 20 mit der Bremsvorrichtung 36 wirkt das hier mittels der Magnetmittel 40 und dem Bremselement 38 erzeugte Bremsmoment MB auch auf die Spindel 20 und über dieses an Betätigungsvorrichtung 16. Auf diese Weise bewegt sich diese während des Infusionsvorganges lediglich mit der am ersten und zweiten Stellelement eingestellten Geschwindigkeit beziehungsweise über die entsprechende Infusionsdauer hinweg.

Dabei wird ein Öffnen des Deckels 6 während der laufenden Infusion durch den in der Sperrstellung stehenden ersten Sperrmechanismus der Sperrvorrichtung 58 verhindert. Zudem werden während der laufenden Infusion, abgesehen von dem Einschaltelement 60, das nötigenfalls in eine Aus-Stellung der Dauerinfusionsvorrichtung 2 verbringbar ist, auch alle sonstigen Stellelemente in der eingestellten Position verriegelt, um ein versehentliches Verstellen während der Infusion zu verhindern.

Regulär wird der Infusionsvorgang dadurch beendet, dass die Betätigungsvorrichtung 16 an dem über das dritte Stellelement 52 positionierten Endanschlag anschlägt, wodurch die Betätigungsvorrichtung 16 nicht weiter durch die Konstantkraftfeder 24 verlagert werden kann. Im Wesentlichen zeitgleich beaufschlagt die Betätigungsvorrichtung auch das Auslöseelement 68, das dabei das Anschlagselement 66 freigibt, das wiederum impulsartig gegen die Glocke 64 beschleunigt wird.

Durch das Erklingen der Glocke 64 wird dem Patienten signalisiert, dass der Infusionsvorgang abgeschlossen ist.

## Patentansprüche

1. Vorrichtung (2) für Dauerinfusionen zur Verabreichung von Medikamenten
mit einem Gehäuse (4), in dem ein Infusionsgerät (10) mit wenigstens einem auspressbaren Infusionsflüssigkeitsbehälter (12) einsetzbar ist,
einer Betätigungsvorrichtung (16) zum Auspressen des Infusionsgerätes (10),
einem Antrieb (18) zur Verlagerung der Betätigungsvorrichtung (16), der eine mechanische Kraftspeichereinrichtung (22) aufweist, und
einer mechanischen Steuerungsanordnung (34), mittels der eine Geschwindigkeit der Betätigungsvorrichtung (16) einstellbar ist,
**dadurch gekennzeichnet, dass** die Steuerungsanordnung (34) eine Bremsvorrichtung (36) aufweist, die wenigstens ein abbremsbares Bremselement (38), das mit dem Antrieb (18) bewegungsgekoppelt ist, und Magnetmittel (40) vorsieht, mittels denen ein Magnetfeld erzeugbar ist, das das Bremselement (38) mit einer Bremskraft (FB) beaufschlagt, wobei die Magnetmittel (40) zur Einstellung der Bremskraft gegenüber dem Bremselement (38) in ihrer Position verstellbar sind.

2. Dauerinfusionsvorrichtung nach Anspruch 1, **dadurch gekennzeichnet, dass** das Bremselement (38) durch eine mittels des Antriebes (18) rotierbare Bremsscheibe gebildet ist.

3. Dauerinfusionsvorrichtung nach Anspruch 1 oder 2, **dadurch gekennzeichnet, dass** die Magnetmittel (40) durch mindestens einen zum Bremselement (38) beabstandet gehaltenen Dauermagnet gebildet sind.

4. Dauerinfusionsvorrichtung nach einem der Ansprüche 1 bis 3, **dadurch gekennzeichnet, dass** die Magnetmittel (40) über ein außerhalb des Gehäuses (4) betätigbares erstes Stellelement (42) verstellbar sind.

5. Dauerinfusionsvorrichtung nach einem der Ansprüche 1 bis 4, **dadurch gekennzeichnet, dass** die Steuerungsanordnung (34) ferner zwischen dem Antrieb (18) und dem Bremselement (38) ein zwischen mindestens zwei unterschiedlichen Übersetzungen schaltbares Getriebe (46) aufweist.

6. Dauerinfusionsvorrichtung nach Anspruch 5, **dadurch gekennzeichnet, dass** das Getriebe (46) durch ein schaltbares Planetengetriebe gebildet ist.

7. Dauerinfusionsvorrichtung nach Anspruch 5 oder 6, **dadurch gekennzeichnet, dass** das Getriebe (46) über ein außerhalb des Gehäuses (4) betätigbares zweites Stellelement (50) umschaltbar ist.

8. Dauerinfusionsvorrichtung nach einem der Ansprüche 1 bis 7, **dadurch gekennzeichnet, dass** die Betätigungsvorrichtung (16) einen Schlitten (21) aufweist, der entlang einer mit dem Bremselement (38) drehgekoppelten Spindel (20) verlagerbar ist und an dem eine mit der Spindel (20) kämmende Spindelmutter (32) sowie ein an einen Stößel (14) des Infusionsgerätes (10) anlegbares Beaufschlagungselement (57) vorgesehen ist.

9. Dauerinfusionsvorrichtung nach Anspruch 8, **dadurch gekennzeichnet, dass** die mechanische Kraftspeichereinrichtung (22) durch eine von Hand spannbare Konstantkraftfeder (24) gebildet ist, die an dem Schlitten (21) angreift.

10. Dauerinfusionsvorrichtung nach einem der Ansprüche 1 bis 9, **dadurch gekennzeichnet, dass** das Infusionsgerät (10) in einer Infusionsgeräteaufnahme (8) gehalten ist, die vor Aktivierung der Betätigungsvorrichtung (16) zum Ausgleich unterschiedlicher Füllmengen des Infusionsflüssigkeitsbehälters (12) gegenüber der Betätigungsvorrichtung (16) verlagerbar ist.

11. Dauerinfusionsvorrichtung nach Anspruch 10, **dadurch gekennzeichnet, dass** das Gehäuse (4) einen zwischen einer Offenstellung und einer Schließstellung verschwenkbaren Deckel (6) aufweist und die Infusionsgeräteaufnahme (8) mittels eines Wegaufnehmers (54) verlagerbar ist, der beim Verschwenken des Deckels (6) in die Schließstellung beaufschlagbar ist.

12. Dauerinfusionsvorrichtung nach Anspruch 10 oder 11, **dadurch gekennzeichnet, dass** ein erster Sperrmechanismus vorgesehen ist, der in Abhängigkeit einer Position der Betätigungsvorrichtung (16) zwischen einer Sperrstellung, in der der Deckel (6) in der Schließstellung verriegelbar ist, und einer Passivstellung verstellbar ist, in der der Deckel (6) von der Schließstellung in die Offenstellung verschwenkbar ist.

13. Dauerinfusionsvorrichtung nach Anspruch 11 oder 12, **dadurch gekennzeichnet, dass** die Betätigungsvorrichtung (16) durch ein von außerhalb des Gehäuses (4) betätigbares Einschaltelement (60) aktivierbar ist und ein zweiter Sperrmechanismus vorgesehen ist, der die Betätigung des Einschaltelementes (60) in der Offenstellung des Deckels (6) blockiert.

14. Dauerinfusionsvorrichtung nach einem der Ansprüche 1 bis 13, **dadurch gekennzeichnet, dass** ein akustischer Signalgeber (62) vorgesehen ist, der am Ende einer Verlagerungsbewegung der Betätigungsvorrichtung (16) durch diese aktivierbar ist.

15. Dauerinfusionsvorrichtung nach Anspruch 14, **dadurch gekennzeichnet, dass** der Signalgeber (62) eine Glocke (64) sowie ein in einer vorgespannten Stellung verriegelbares und gegen die Glocke (64) impulsartig verlagerbares Anschlagelement (66) aufweist, wobei die Verriegelung mittels der Betätigungsvorrichtung (16) lösbar ist.

16. Dauerinfusionsvorrichtung nach Anspruch 14, **dadurch gekennzeichnet, dass** der Signalgeber (62) durch einen elektronischen Signalgeber gebildet ist, der mit einem über einen Generator aufladbaren Energiespeicher verbunden ist, mittels dem der Antrieb (18) wenigstens teilweise abbremsbar ist.

17. Dauerinfusionsvorrichtung nach Anspruch 16, **dadurch gekennzeichnet, dass** die Bremsvorrichtung (36) durch den Generator gebildet ist.

## Claims

1. Device (2) for continuous infusion for the administration of medications,
comprising a housing (4), in which an infusion device (10) can be inserted, with at least one infusion liquid container (12) which can be pressed out,
an actuating device (16) for pressing out the infusion device (10),
a drive unit (18) for repositioning the actuating device (16), which comprises a mechanical energy storage device (22), and
a mechanical control arrangement (34), by means of which the speed of the actuating device (16) can be adjusted,
**characterised in that** the control arrangement (34) comprises a brake device (36), which provides at least one brake element (38) which can be braked, which is movement-coupled to the drive unit (18), and magnetic means (40), by means of which a magnetic field can be generated, which imposes a braking force (FB) on the brake element (38), wherein the magnetic means (40) can be adjusted in order to adjust the braking force in relation to the braking element (38) in its position.

2. Continuous infusion device in accordance with claim 1, **characterised in that** the brake element (38) is formed by a brake disk which can be rotated by means of the drive unit (18).

3. Continuous infusion device in accordance with claim 1 or 2, **characterised in that** the magnetic means (40) are formed by at least one permanent magnet, held at a distance interval from the brake element (38).

4. Continuous infusion device in accordance with any one of claims 1 to 3, **characterised in that** the magnetic means (40) can be adjusted by a first positioning element (42) which can be actuated from outside the housing (4).

5. Continuous infusion device in accordance with any one of claims 1 to 4, **characterised in that** the control arrangement (34) further comprises, between the drive (18) and the brake element (38), a gear unit (46) which can be switched between at least two different transmission ratios.

6. Continuous infusion device in accordance with claim 5, **characterised in that** the gear unit (46) is formed by a switchable planetary gear arrangement.

7. Continuous infusion device in accordance with claim 5 or 6, **characterised in that** the gear unit (46) can be switched over by means of a second positioning element (50) which can be actuated from outside the housing (4).

8. Continuous infusion device in accordance with any one of claims 1 to 7, **characterised in that** the actuating device (16) comprises a carriage (21), which can be moved along a spindle (20), rotation-coupled to the brake element (38), and provided at which is a spindle nut (32), which meshes with the spindle (20), as well as a force imposition element (57) which can be imposed on a plunger (14) of the infusion device (10).

9. Continuous infusion device in accordance with claim 8, **characterised in that** the mechanical energy storage device (22) is formed by a constant force spring (24) which can be tensioned by hand, and which engages on the carriage (21).

10. Continuous infusion device in accordance with any one of claims 1 to 9, **characterised in that** the infusion device (10) is held in a diffusion device receiver (8), which, before the activating of the actuating device (16), can be moved in relation to the actuating device (16) in order to compensate for different filling quantities of the infusion liquid container (12).

11. Continuous infusion device in accordance with claim 10, **characterised in that** the housing (4) comprises a cover (6) which can be pivoted between an open position and a closed position, and the infusion device receiver (8) can be moved by means of a transducer (54), which can be subjected to force when the cover (6) is pivoted into the closed position.

12. Continuous infusion device in accordance with claim 10 or 11, **characterised in that** a first blocking mechanism is provided, which, depending on the position of the actuating device (16), can be adjusted between a blocking position, in which the cover (6) can be locked in the closed position, and a passive position, in which the cover (6) can be pivoted from the closed position into the open position.

13. Continuous infusion device in accordance with claim 11 or 12, **characterised in that** the actuating device (16) can be activated by a switching element (60) which can be actuated from outside the housing (4), and a second blocking mechanism is provided, which blocks the actuation of the switching element (60) in the open position of the cover (6).

14. Continuous infusion device in accordance with any one of claims 1 to 13, **characterised in that** an acoustic signal generator (62) is provided, which, at the end of the displacement movement of the actuating device (16) can be deactivated by this device.

15. Continuous infusion device in accordance with claim 14, **characterised in that** the signal generator (62) comprises a bell (64) and a striker element (66), which can be locked in a pre-tension position, and can be moved against the bell (64) in pulsed fashion, wherein the locking effect can be released by the actuating device (16).

16. Continuous infusion device in accordance with claim 14, **characterised in that** the signal generator (62) is formed by an electronic signal generator, which is connected to an energy storage device which can be charged by a generator, by means of which the drive unit (18) can at least partially be braked.

17. Continuous infusion device according to claim 16, **characterised in that** the braking device (36) is formed by the generator.

## Revendications

1. Dispositif (2) de perfusion continue pour administrer des médicaments
comprenant un boîtier (4), dans lequel peut être inséré un appareil de perfusion (10) comprenant au moins un récipient pour liquide de perfusion (12) pouvant être éjecté,
un dispositif d'actionnement (16) pour l'éjection de l'appareil de perfusion (10),
un système d'entraînement (18) pour le déplacement du dispositif d'actionnement (16) comprenant un dispositif accumulateur d'énergie mécanique (22), et
un agencement de commande mécanique (34), au moyen duquel une vitesse du dispositif d'actionnement (16) peut être réglée,
**caractérisé en ce que** l'agencement de commande (34) comprend un dispositif de freinage (36), comportant au moins un élément de freinage (38) pouvant être freiné, qui est accouplé, solidaire en déplacement, avec le système d'entraînement (18), et des moyens magnétiques (40), à l'aide desquels un champ magnétique peut être produit, de manière à solliciter l'élément de freinage (38) par une force de freinage (FB), les moyens magnétiques (40) pouvant être réglés en position pour ajuster la force de freinage par rapport à l'élément de freinage (38).

2. Dispositif de perfusion continue selon la revendication 1, **caractérisé en ce que** l'élément de freinage (38) est formé par un disque de frein pouvant être rotatif au moyen du système d'entraînement (18).

3. Dispositif de perfusion continue selon la revendication 1 ou 2, **caractérisé en ce que** les moyens magnétiques (40) sont formés par au moins un aimant permanent maintenu espacé par rapport à l'élément de freinage (38).

4. Dispositif de perfusion continue selon l'une des revendications 1 à 3,
**caractérisé en ce que** les moyens magnétiques (40) peuvent être réglés par l'intermédiaire d'un premier élément de réglage (42) pouvant être actionné à l'extérieur du boîtier (4).

5. Dispositif de perfusion continue selon l'une des revendications 1 à 4,
**caractérisé en ce que** l'agencement de commande (34) comprend en outre entre le système d'entraînement (18) et l'élément de freinage (38) une transmission (46) pouvant être commutée entre au moins deux rapport de transmission différents.

6. Dispositif de perfusion continue selon la revendication 5, **caractérisé en ce que** la transmission (46) est formée par un train épicycloïdal commutable.

7. Dispositif de perfusion continue selon la revendication 5 ou 6, **caractérisé en ce que** la transmission (46) peut être commutée par l'intermédiaire d'un deuxième élément de réglage (50) pouvant être actionné à l'extérieur du boîtier (4).

8. Dispositif de perfusion continue selon l'une des revendications 1 à 7,
**caractérisé en ce que** le dispositif d'actionnement (16) comprend un chariot (21) qui est déplaçable le long d'une tige filetée (20) accouplée solidaire en rotation avec l'élément de freinage (38) et sur laquelle est prévu un écrou de tige filetée (32) engrenant avec la tige filetée (20) ainsi qu'un élément d'appui (57) pouvant être appliqué sur un poussoir (14) de l'appareil de perfusion (10).

9. Dispositif de perfusion continue selon la revendication 8, **caractérisé en ce que** le dispositif accumulateur d'énergie mécanique (22) est formé par un ressort à force constante (24) pouvant être tendu manuellement et coopérant avec le chariot (21).

10. Dispositif de perfusion continue selon l'une des revendications 1 à 9,
**caractérisé en ce que** l'appareil de perfusion (10) est maintenu dans un réceptacle de l'appareil de perfusion (8) qui est déplaçable par rapport au dispositif d'actionnement (16) avant l'activation du dispositif d'actionnement (16) pour compenser les différentes quantités du récipient pour liquide de perfusion (12).

11. Dispositif de perfusion continue selon la revendication 10, **caractérisé en**
**ce que** le boîtier (4) comprend un couvercle (6) pouvant pivoter entre une position d'ouverture et une position de fermeture et le réceptacle de l'appareil de perfusion (8) est déplaçable au moyen d'un capteur de déplacement (54) qui peut être sollicité lors du pivotement du couvercle (6) dans la position de fermeture.

12. Dispositif de perfusion continue selon la revendication 10 ou 11,
**caractérisé en ce qu'**il est prévu un premier mécanisme de verrouillage pouvant être réglé en fonction d'une position du dispositif d'actionnement (16) entre une position de verrouillage, dans laquelle le couvercle (6) est verrouillable dans la position de fermeture, et une position passive, dans laquelle le couvercle (6) peut être pivoté depuis la position de fermeture à la position d'ouverture.

13. Dispositif de perfusion continue selon la revendication 11 ou 12,
**caractérisé en ce que** le dispositif d'actionnement (16) peut être activé par un élément de commutation (60) pouvant être actionné depuis l'extérieur du boîtier (4) et il est prévu un deuxième mécanisme de verrouillage bloquant l'actionnement de l'élément de commutation (60) dans la position d'ouverture du couvercle (6).

14. Dispositif de perfusion continue selon l'une des revendications 1 à 13,
**caractérisé en ce qu'**il est prévu un émetteur de signaux acoustiques (62) pouvant être activé par le dispositif d'actionnement (16) à la fin d'un mouvement de déplacement de celui-ci.

15. Dispositif de perfusion continue selon la revendication 14, **caractérisé en ce que** l'émetteur de signaux (62) comprend une cloche (64) ainsi qu'un élément de butée (66) pouvant être déplacé par impulsion contre la cloche (64) et pouvant être verrouillé dans une position précontrainte, le verrouillage étant amovible au moyen du dispositif d'actionnement (16).

16. Dispositif de perfusion continue selon la revendication 14, **caractérisé en ce que** l'émetteur de signaux (62) est formé par un émetteur de signaux électronique qui est connecté à une réservoir d'énergie pouvant être chargé par un générateur, réservoir au moyen duquel le système d'entraînement (18) peut être freiné au moins partiellement.

17. Dispositif de perfusion continue selon la revendication 16, **caractérisé en ce que** le dispositif de freinage (36) est formé par le générateur.
